Europäisches Patentamt

European Patent Office

Office européen des brevets

(11) Publication number: **0 448 840 A2**

# EUROPEAN PATENT APPLICATION

(21) Application number: 90125805.3

(22) Date of filing: **29.12.90**

(51) Int. Cl.5: **A61L 31/00**, A61L 17/00, A61L 27/00

(30) Priority: **26.02.90 US 484318**

(43) Date of publication of application:
**02.10.91 Bulletin 91/40**

(84) Designated Contracting States:
**BE CH DE DK ES FR GB GR IT LI NL SE**

(71) Applicant: **AMERICAN CYANAMID COMPANY**
**1937 West Main Street P.O. Box 60**
**Stamford Connecticut 06904-0060(US)**

(72) Inventor: **Bradley, Douglas P.**
**9 Tommy's Lane**
**Brookfield, State of Connecticut 06904(US)**

(74) Representative: **Wächtershäuser, Günter, Dr.**
**Tal 29**
**W-8000 München 2(DE)**

(54) Bicomponent or blended wound closure device.

(57) The wound closure device is manufactured from a bicomponent surgical filament comprising a core and a sheath. The core can be extruded from polyethylene terephthalate or polypropylene. The sheath can be selected from polybutylene terephthalate, polybutester, or nylon. Alternatively, the filament can be a blend of polyesters selected from two or more of polyethylene terephthalate or polybutylene terephthalate, and polybutester. The wound closure device can be a tissue repair device, vascular graft, or a surgical mesh or suture.

EP 0 448 840 A2

The following polymeric materials have been used to manufacture wound closure devices: polybutylene terephthalate ("PBT"), polyethylene terephthalate ("PET"), polybutester (e.g., Hytrel™ polybutester, du Pont, DE, USA), polypropylene ("PP") and nylon (e.g., nylon 66 and nylon 6). Some, if not all, of these materials have a disadvantage when used as the sole component in a wound closure device, for example a suture in a cardiovascular ("CV") surgical procedure. The major drawback to polypropylene may be its tendency to fray during knot rundown. PET may be too stiff when used as a monofilament. Nylon is thought to lose strength in-vivo. This may make it unsuitable for permanent CV applications.

Since none of the above materials appear to be ideal when used above to manufacture a wound closure device, this invention combines at least two of the materials to trade off or balance-out their respective individual weaknesses as a surgical product. The invention is achieved by fabricating a bicomponent (sheath and core) fiber or by blending two or more polymers together in extrusion.

An outline of the bicomponent approach is as follows:

| | | Sheath | |
|---|---|---|---|
| | PBT | Polybutester | Nylon |
| Core | | | |
| PET | X | X | X |
| PP | X | X | X |

As shown in the outline, this invention uses either PP or PET as the core material and either PBT, polybutester or nylon as the sheath material. Because PP and polybutester are known from the prior art to be useful as a surgical suture, and more specifically, to be useful in CV surgical procedures, they have been exemplified. Also, the ease of knot rundown of a PP suture may be improved if the sheath of the bicomponent surgical filament is a material other than PP. For example, it may be possible to manufacture a bicomponent filament having a PP core and a polybutester sheath, wherein the bicomponent filament has the advantageous knot rundown properties of a polybutester suture (that is, the Novafil™ suture, Davis & Geck, CT, USA) and the relatively low tissue drag of a polypropylene suture. Of approximately equal interest is a PP core and PBT sheath material. PBT is known from the prior art to be useful as a surgical suture.

## Description

A bicomponent surgical filament useful in a wound closure device has been invented. The bicomponent surgical filament comprises a core having a polymer selected from the group consisting of polyethylene terephthalate and polypropylene, and a sheath comprising a polymer selected from the group consisting of polybutylene terephthalate, polybutester and nylon. In one embodiment, the core is polypropylene. In a specific embodiment, the sheath is polybutylene terephthalate or polybutester. In a more specific embodiment, the sheath is polybutester.

In another embodiment, the bicomponent surgical filament is braided into a tissue repair device. In a specific embodiment, the braided device is a ligament or tendon repair device. In yet another embodiment, the filament is knitted into a vascular graft or surgical mesh. In still another embodiment, the filament is woven into a vascular graft.

A surgical filament comprising a blend of polyesters and useful in a wound closure device has also been invented. The polyesters are selected from the group consisting of polyethylene terephthalate, polybutylene terephthalate and polybutester. In one embodiment, the polyester is selected from polyethylene terephthalate and polybutylene terephthalate. In another embodiment, the blend consists of polyethylene terephthalate and polybutester. In still another embodiment, the blend consists of polybutylene terephthalate and polybutester.

In yet another embodiment, the surgical filament comprising a blend of polyesters is braided into a tissue repair device. In a specific embodiment, the braided device is a ligament or tendon repair device. In still yet another embodiment, the filament is knitted into a vascular graft or surgical mesh. In a further embodiment, the filament is woven into a vascular graft.

A wound closure device manufactured from a bicomponent surgical filament has been invented. The core of the bicomponent surgical filament consists of a polymer selected from the group consisting of polyethylene terephthalate and polypropylene, and a sheath selected from a polyester consisting of polybutylene terephthalate, polybutester, and blends of the same. In one embodiment, the core is polypropylene. In a specific embodiment the sheath is polybutylene terephthalate or polybutester. In another specific embodiment, the sheath is a blend of polybutylene terephthalate and polybutester. In a more specific embodiment, the wound closure device is a surgical suture.

A drawing which describes the shape and/or geometrical configuration of the bicomponent surgical filament or of the filament comprising a blend of polyesters is not necessary for an understanding of this invention. That is, any person skilled in the surgical filament art will know how to manufacture and how to use the invention by reading this specification generally, and the examples specifically.

The PP/polybutester bicomponent fiber may have to overcome certain problems during processing, for example the difference in extensibilities of each polymer during drawing. Also, it is not known if this PP/Polybutester bicomponent fiber will have less tissue drag than a polybutester surgical suture (e.g. the NovafilTM suture, Davis & Geck, CT, USA) in a CV application.

As a blend, only the above polymers that are polyesters appear to be useful. This is because only the polyesters exhibit any degree of miscibility in each other. Of these, blends of PET and PBT, and blends of PET and polybutester appear to be the most useful. It is intended that the PET portion of the blend will be used to stiffen the surgical filament, which in turn will reduce or even eliminate in-vivo tissue drag. However, blends of PBT and polybutester, and blends of PET, PBT and polybutester are within the scope of this invention.

Also within the scope of this invention is a bicomponent surgical filament wherein either or both the core and the sheath is a blend of two or more polyesters, with the proviso that if both the core and the sheath are blends, they will be of either different compositions or proportions. For example, a core of PET and a sheath of PET and PBT is within the scope of this invention.

Example 1

A two-component coextrusion system was configured to produce fiber with the two polymeric phases in a concentric sheath and core arrangement. The core material was extruded from a MoplenTM polypropylene resin (Aristech Corp., WV, USA) through a single screw extruder at a rate of approximately 1.2 lb/hr and a temperature of about 200 to 210°C. The sheath component was concurrently extruded from a HytrelTM polybutester (du Pont, DE, USA) through a second single screw extruder at a rate of approximately 2.0 lb/hr and a temperature of approximately 240°C. Both materials were extruded into a bicomponent die that formed a single extrudate strand with the two materials in distinct phases with concentric circular cross-sections. The strand was then cooled by water quenching and wound onto a spool at a speed of 165 ft/min.

Example 2

The extrudate of Example 1 was then drawn continuously in two stages, with a 4x draw in the first stage and a 1.4x draw in the second stage. The temperature in both stages was about 160°C. The physical properties of the resulting fiber were as follows:

| Diameter (mm) | Straight-Pull Strength (kg) | Knot-Pull Strength (kg) | Breaking Elongation (%) |
|---|---|---|---|
| 0.348 | 3.55 | 2.24 | 21 |

Example 3

The fiber of Example 2 was then cut into lengths, attached with needles, and sterilized by exposure to ethylene oxide. The cutting, attaching and sterilizing were by known prior art methods. The physical properties of the resulting surgical suture were as follows:

|  | Diameter (mm) | Straight-Pull Strength (kg) | Knot-Pull Strength (kg) | Breaking Elongation (%) |
|---|---|---|---|---|
|  | 0.383 | 3.52 | 2.66 | 26 |

## Example 4

A bicomponent fiber extrusion system was configured similar to Example 1. The core material was extruded from a polypropylene resin at rates of approximately 0.10 to 0.16 lb/hr and a temperature of about 210 to 220°C. The sheath component was extruded from Hytrel™ polybutester (du Pont, DE, USA) at rates of approximately 0.15 to 0.20 lb/hr and temperatures of about 230°C. The resulting single strand was cooled by air quenching and collected at speeds of approximately 160 to 180 ft/min.

## Example 5

The extrudate of Example 4 was then drawn continuously in a single step with a draw ratio 5.75x at a temperature of approximately 80°C. The physical properties of the resulting fiber were as follows:

|  | Diameter (mm) | Straight-Pull Strength (kg) | Knot-Pull Strength (kg) | Breaking Elongation (%) |
|---|---|---|---|---|
|  | 0.095 | 0.260 | 0.200 | 31 |

## Example 6

The fiber of Example 5 was then cut into lengths, attached with needles, and sterilized by exposure to ethylene oxide. The cutting, attaching and sterilizing were by methods essentially identical to those of Example 3. The physical properties of the resulting surgical suture were as follows:

|  | Diameter (mm) | Straight-Pull Strength (kg) | Knot-Pull Strength (kg) | Breaking Elongation (%) |
|---|---|---|---|---|
|  | 0.081 | 0.230 | 0.210 | 25 |

## Example 7

The following sutures were evaluated in vivo:

4

| Suture | Suture Description |
|---|---|
| 1. | 6/0 PP/Hytrel™ Bicomponent |
| 2. | 6/0 PBT (homopolymer from GAF Corp., USA), cold drawn @ $60^{\circ}$C |
| 3. | 6/0 PVDF-HFP copolymer (from Solef™ 11010-000 resin, Soltex, Inc., Texas, USA) |
| 4. | 6/0 PP (SURGILENE™, Davis & Geck, USA) |

Suture 1. is the same as or similar to the surgical suture described in Example 6. All sutures were double armed with TE-1 needles. In suture 3., "PVDF-HFP" is an abbreviation for vinylidene fluoride-co-hexafluoro propylene polymer.

Each suture was used once by each of two investigators and twice by a third investigator for anastomosis of the carotid or femoral artery in two dogs that had just been sacrificed.

Eight anastomoses were done in the carotid and femoral arteries of a beagle, and eight were done in the carotid arteries of a foxhound. The open, or "parachute" technique was used in each case. In this technique, the back wall of the anastomosis is stitched while the vessel ends are kept 1-2 cm apart. Then the vessel ends are drawn together and the suture tightened by pulling on the two suture ends. The front half of the anastomosis is completed by tying a standard knot. Sutures were evaluated subjectively for smoothness (ease of "pull-up") and overall handling properties.

For purposes of summarizing the data, tissue drag and handling for each trial were rated " + " or "-". These ratings and the total score for each suture are given in Table 1.

## Table 1

Summary of Tissue Drag (Suture "Pull-Up") and Handling Property Evaluation of Sutures in Open Arterial Anastomoses.

| | Tissue Drag | | | | Handling | | | | Total |
|---|---|---|---|---|---|---|---|---|---|
| | 4-6 Loops | | 7-9 Loops | | | | | | |
| Trial No.: | 1 | 2 | 1 | 2 | 1 | 2 | 3 | 4 | Score |
| **Suture** | | | | | | | | | |
| 1. Bicomponent | + | + | + | − | + | − | ± | − | 3.5 |
| 2. PBT/Cold Draw | + | + | + | + | + | + | ± | + | 7.5 |
| 3. PVDF/HFP | + | + | − | + | + | + | ± | + | 6.5 |
| 4. Surgilene™ | − | + | − | + | ± | + | + | + | 5.5 |

+ = Good pull-up/handling

− = Resistance to pull-up/difficult handling

Example 8

The comments made by the three investigators during the anastomoses of Example 7 for the bicomponent and PBT (polybutylene terephthalate) sutures are shown in Table 2 below. All sutures pulled up satisfactorily when 4-6 loops were used (2 trials/suture). With 7-9 loops, only PBT drew up easily in both trials. All others showed some resistance in one of the two trials. In the case of the bicomponent, this resistance may have been due to the excessive curling, which caused the suture to catch on itself. In the case of the SurgileneTM suture, the resistance may have been due to a knot in the suture, discovered after the suture ends were drawn up.

## Table 2

### Tissue Drag and Handling Evaluation of Four Cardiovascular Sutures

| Suture Material | Opera-tor | Site | No. of Loops | Comments |
|---|---|---|---|---|
| 1. Bicom-ponent | 1. | R. carotid – beagle | 4 | Very smooth pull-up. Must maintain tension on suture to prevent rebound. Too curly. |
| | 2. | L. Carotid – beagle | 4 | Same as above. Color very pale. |
| | 3. | R. carotid – foxhound | 7 | Draws up very nicely. Ties nicely. Stretchy. Difficult to see. |
| | 1. | L. carotid – foxhound | 9 | Noticeable tissue drag. Pulled up with difficulty. Curly, difficult to handle. |
| 2. PBT Cold Draw | 1. | R. carotid – beagle | 6 | Nice smooth pull-up. Handles better than previous suture. A very nice suture. |
| | 2. | L. carotid – beagle | 6 | Nice smooth pull-up. Rebound not a problem. Prefer to previous suture. |
| | 3. | R. carotid – foxhound | 8 | Draws up well. Curly. Prefers over other 3 sutures. |
| | 1. | L. carotid – foxhound | 8 | Pulls up very smoothly. Little resistance. Handles well. |

Summary and Conclusions - Examples 7 and 8

The tissue drag and handling properties of the four sutures were evaluated in arterial anastomoses in the dog. Each suture was used a total of four times by three different operators. Based on subjective ratings of tissue drag (i.e., ease of drawing up 4-9 suture loops in an open anastomosis) and general handling properties, the preferred suture was PBT/Cold Draw, followed by PVDF/HFP. The bicomponent suture was rated lowest due to its extreme curliness.

**Claims**

1. A wound closure device manufactured from a first bicomponent filament or a second filament characterized by a blend of polyesters, the first bicomponent filament characterized by a core having a polymer selected from the group consisting of polyethylene terephthalate and polypropylene, and a sheath comprising a polyester or nylon, the polyester selected from the group consisting of polybutylene terephthalate, polybutester and blends of the same; and the blend of polyesters in the second filament selected from the group consisting of polyethylene terephthalate, polybutylene terephthalate and polybutester.

2. The device of claim 1 wherein the core of the first filament is polypropylene.

3. The device of claim 2 wherein the sheath of said first filament is polybutylene terephthalate and/or polybutester.

4. The device of claim 3 wherein the sheath is polybutester.

5. The device of claim 1 wherein said blend of polyesters in said second filament consists essentially of polyethylene terephthalate and polybutylene terephthalate.

6. The device of claim 1 wherein said blend of polyesters in said second filament consists essentially of polyethylene terephthalate and polybutester.

7. The device of claim 1 to 6 wherein said first bicomponent or second filament is braided into a tissue repair device.

8. The device of claim 1 to 6 wherein said first bicomponent or second filament is knitted or woven into a vascular graft.

9. The device of claim 1 to 6 wherein said first bicomponent or second filament is knitted into a surgical mesh.

10. The device of claim 1 to 6 wherein said device is a surgical suture.